# EUROPÄISCHE PATENTANMELDUNG

(11) **EP 2 565 624 A1**
(43) Veröffentlichungstag der Anmeldung: **06.03.2013**
(21) Anmeldenummer: 11007168.5
(22) Anmeldetag: 05.09.2011
(51) Int. Cl.: G01N 21/35, G01N 21/64

(54) **Sensorkopf zur Verwendung bei transkutanen Organfunktionsmessungen**

(71) Anmelder: Carl Freudenberg KG, 69469 Weinheim (DE); Universität Heidelberg, 69117 Heidelberg (DE)
(72) Erfinder: Die Erfindernennung liegt noch nicht vor

(57) **Zusammenfassung**

Ein Sensorkopf, umfassend ein Gehäuse (1) mit einer Anlagefläche (2), welche einem menschlichen oder einem tierischen Körper (3) zuwendbar ist, mit mindestens einer Abfragelichtquelle (4) und mit einem Detektor (5), wobei der Detektor (5) Antwortlicht (6) detektiert, welches durch die Abfragelichtquelle (4) erzeugbar ist, ist im Hinblick auf die Aufgabe, einen Sensorkopf anzugeben, welcher bei flachem und einfachem Aufbau Organfunktionen des menschlichen Körpers zuverlässig erfassen kann, dadurch gekennzeichnet, dass die Abfragelichtquelle (4) und der Detektor (5) auf oder in einem ebenen Bereich (7a) einer Leiterplatte (7) angeordnet sind, wobei sich der Bereich (7a) der Leiterplatte (7) auf oder im Gehäuse (1) erstreckt

## Beschreibung

### Technisches Gebiet

Die Erfindung betrifft einen Sensorkopf gemäß dem Oberbegriff des Patentanspruchs 1.

### Stand der Technik

Aus der WO 2010/ 020 673 A2 ist ein Sensor bekannt, welcher verwendet wird, um die Funktion und Leistung von Organen im menschlichen Körper zu erfassen.

Hierzu werden in den menschlichen Körper fluoreszierende Flüssigkeiten, genannt Marker, injiziert, die von den zu untersuchenden Organen des Körpers eliminiert werden. Anhand der Eliminationsrate des Markers kann die Vitalität und Funktion der Organe überwacht werden.

Die Eliminationsrate wird ermittelt, indem Abfragelicht in den Körper eingestrahlt und Antwortlicht, nämlich Fluoreszenzlicht, detektiert wird. Das Abfragelicht regt einen fluoreszierenden Stoff im Körper zur Abgabe von Fluoreszenzlicht an.

Je nach der Intensität des Fluoreszenzlichts kann auf die im Körper noch vorhandene Menge des fluoreszierenden Stoffs und damit die Leistung bzw. den Zustand des Organs geschlossen werden. Nicht invasive Messungen dieser Art werden als transkutane Messungen bezeichnet. Bei diesen Messungen wird durch die Haut hindurch gemessen.

Aus dem Gebrauchsmuster DE 20 2010 014 729 U1 ist ein Sensorkopf mit einem relativ hohen Gehäuse aus Kunststoff bekannt. Dieser Sensorkopf weist eine Photodiode und zwei LEDs auf, die Licht mit unterschiedlichen Wellenlängen abstrahlen. Von den LEDs wird Licht derart abgestrahlt, dass Einflüsse von Fremdlicht und Reflexionen kompensiert werden können. Die beiden LEDs und die Photodiode sind vor diesem Hintergrund relativ zueinander geneigt angeordnet. Die geneigte Anordnung führt zu einem hohen und relativ dicken Aufbau des Sensorkopfs.

Die geneigte Anordnung der LEDs und der Photodiode am bzw. im Sensorkopf führt zu Montageproblemen, wenn nämlich die LEDs und die Photodiode auf einer Leiterplatte montiert sind. Die Leiterplatte muss bei der Anordnung der Photodiode und der LEDs verbogen oder verwunden werden, um die Neigung der Photodiode und der LEDs herzustellen. Hierbei kann die Leiterplatte Schaden nehmen. Des Weiteren können intrinsische Spannungen in der Leiterplatte zu Rückstellmomenten führen, welche die gesamte optische Anordnung stören.

### Darstellung der Erfindung

Der Erfindung liegt daher die Aufgabe zu Grunde, einen Sensorkopf anzugeben, welcher bei flachem und einfachem Aufbau Organfunktionen des menschlichen Körpers zuverlässig erfassen kann.

Erfindungsgemäß wird die voranstehende Aufgabe mit den Merkmalen des Patentanspruchs 1 gelöst.

Ein erfindungsgemäßer Sensorkopf umfasst ein Gehäuse mit einer Anlagefläche, welche einem menschlichen oder einem tierischen Körper zuwendbar ist, mit mindestens einer Abfragelichtquelle und mit einem Detektor, wobei der Detektor Antwortlicht detektiert, welches durch die Abfragelichtquelle erzeugbar ist.

Erfindungsgemäß ist es vorgesehen, dass die Abfragelichtquelle und der Detektor auf oder in einem im Wesentlichen ebenen oder vollständig ebenen Bereich einer Leiterplatte angeordnet sind, wobei sich der Bereich der Leiterplatte auf oder im Gehäuse erstreckt.

Erfindungsgemäß ist zunächst erkannt worden, dass eine geneigte Anordnung der Abfragelichtquellen und des Detektors relativ zueinander zu einem relativ sperrigen und hohen Aufbau des Sensorkopfs führt. Weiter ist erkannt worden, dass die Abfragelichtquelle und der Detektor bisher auf einer flexiblen Leiterplatte montiert werden mussten, um relativ zueinander neigbar zu sein. Erfindungsgemäß ist dann erkannt worden, dass die Abstrahlflächen der Abfragelichtquellen im Wesentlichen parallel zu der Detektionsfläche des Detektors orientiert werden können, ohne Messverluste hinnehmen zu müssen. Die Abfragelichtquellen und der Detektor liegen erfindungsgemäß auf oder in der Ebene einer im Wesentlichen flachen und ebenen Leiterplatte- So kann ein flach bauender Sensorkopf realisiert werden. Darüber hinaus können die Abfragelichtquellen und der Detektor lagestabil im oder auf dem Gehäuse montiert werden. Insoweit ist ein Sensorkopf angegeben, welcher bei flachem und einfachem Aufbau Organfunktionen des menschlichen Körpers zuverlässig erfassen kann.

Folglich ist die eingangs genannte Aufgabe gelöst.

Die Abfragelichtquelle könnte als im Infraroten leuchtende Diode, nämlich als LED, ausgestaltet sein. Überraschend ist erkannt worden, dass Infrarotlicht weit tiefer in die menschliche oder tierische Haut bzw. in das Gewebe eindringt als sichtbares Licht.

Vor diesem Hintergrund könnten zwei Abfragelichtquellen vorgesehen sein, welche als im Infraroten leuchtende Dioden, nämlich als LEDs, ausgestaltet sind, zwischen welchen der Detektor angeordnet ist. Im Gegensatz zum Stand der Technik gemäß der DE 20 2010 014 729 U1 ist keine Referenzlichtquelle notwendig, welche das Signal-zu-Rausch-Verhältnis zwischen Abfragelichtquelle und Antwortlicht verbessert Es können zwei LEDs vorgesehen sein, welche beide einen Marker anregen, Fluoreszenzlicht in Richtung des Detektors abzustrahlen.

Der Detektor könnte als infrarotselektive Photodiode ausgestaltet sein. Überraschend wird als Fluoreszenzlicht Infrarotlicht mit ausreichender Intensität abgestrahlt. Das eingestrahlte Infrarotlicht der Abfragelichtquellen wird nahezu nicht an der Oberfläche der menschlichen oder tierischen Haut reflektiert. Vielmehr dringt es relativ tief in das Gewebe ein und regt den Marker an, als Fluoreszenzlicht ebenfalls Infrarotlicht zu emittieren.

Der Detektor und/ oder die Abfragelichtquellen könnten bzw. könnte als nichtorganische oder organische optische Elemente bzw. nichtorganisches oder organisches optisches Element ausgestaltet sein. Nichtorganische optische Elemente sind relativ kostengünstig.

Die Leiterplatte könnte als starre Leiterplatte ausgestaltet sein. Eine starre Leiterplatte bewirkt eine dauerhaft stabile Anordnung der optischen Elemente, so dass der Strahlengang während des Einsatzes des Sensorkopfes nahezu nicht verändert wird.

Eine oder mehrere Abfragelichtquelle(n) könnte bzw. könnten Anregelicht mit einer Wellenlänge aus dem Bereich 550 nm bis 1400 nm abstrahlen. Überraschend ist erkannt worden, dass Infrarotlicht weit tiefer in die menschliche oder tierische Haut bzw. in das Gewebe eindringt als sichtbares Licht

Vor diesem Hintergrund könnte der Detektor Antwortlicht mit einer Wellenlänge aus dem Bereich 550 nm bis 1400 nm detektieren. Das vom Marker emittierte Fluoreszenzlicht zeigt in diesem Wellenlängenbereich eine besonders hohe Intensität.

Dem Detektor könnte ein Filter vorgeschaltet sein, welcher Primärlicht der Abfragelichtquelle ausblendet. Hierdurch wird nur Antwortlicht, nämlich Fluoreszenzlicht des Markers vom Detektor erfasst.

Ein Pflaster könnte einen Sensorkopf der hier beschriebenen Art umfassen. Hierdurch kann der Sensorkopf problemlos auf den menschlichen oder tierischen Körper aufgeklebt oder aufgebunden werden und ambulant getragen werden. Die transkutanen Messungen sind daher zuverlässig nicht nur im Labor, sondern zu Hause und auch im Alltag mobil durchführbar.

### Kurzbeschreibung der Zeichnung

In der Zeichnung zeigen
- Fig. 1: eine Schnittansicht des Sensorkopfs nebst Strahlengang der Lichtquellen,
- Fig. 2: eine weitere Schnittansicht des Sensorkopfs gemäß Fig. 1,
- Fig. 3: eine perspektivische Ansicht des Sensorkopfs gemäß Fig. 1 und 2 nebst einer Leiterplatte von oben und
- Fig. 4: eine perspektivische Ansicht des Sensorkopfs gemäß Fig. 1 und 2 nebst einer Leiterplatte von unten.

### Ausführung der Erfindung

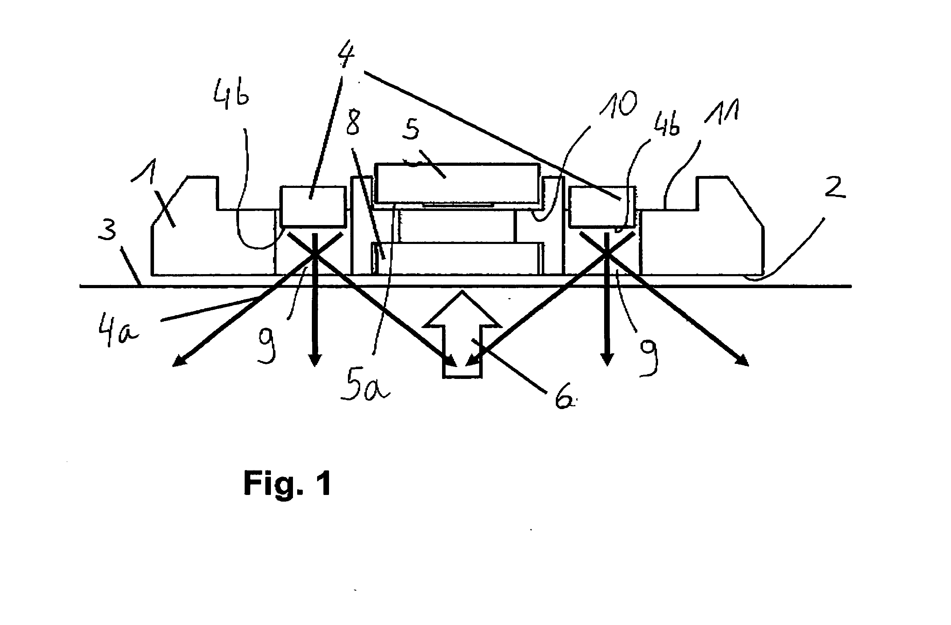

Fig. 1 zeigt einen Sensorkopf, umfassend ein Gehäuse 1 mit einer Anlagefläche 2, welche einem menschlichen oder einem tierischen Körper 3 zuwendbar ist, mit mindestens einer Abfragelichtquelle 4 und mit einem Detektor 5, wobei der Detektor 5 Antwortlicht 6 detektiert, welches durch die Abfragelichtquelle 4 erzeugbar ist. Das Antwortlicht 6 ist Fluoreszenzlicht eines fluoreszierenden Stoffs eines Markers im menschlichen oder tierischen Körper 3. Das Fluoreszenzlicht wird durch Anregelicht 4a einer Abfragelichtquelle 4 induziert.

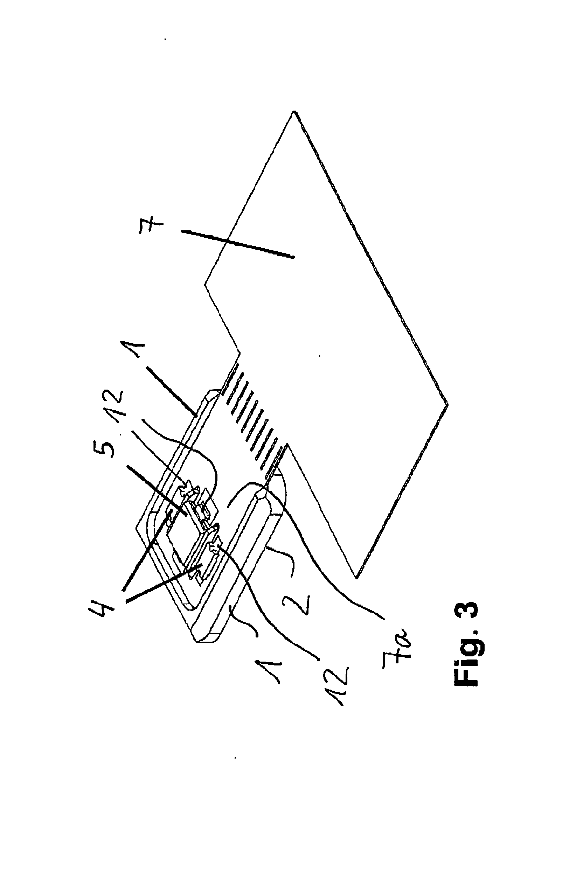

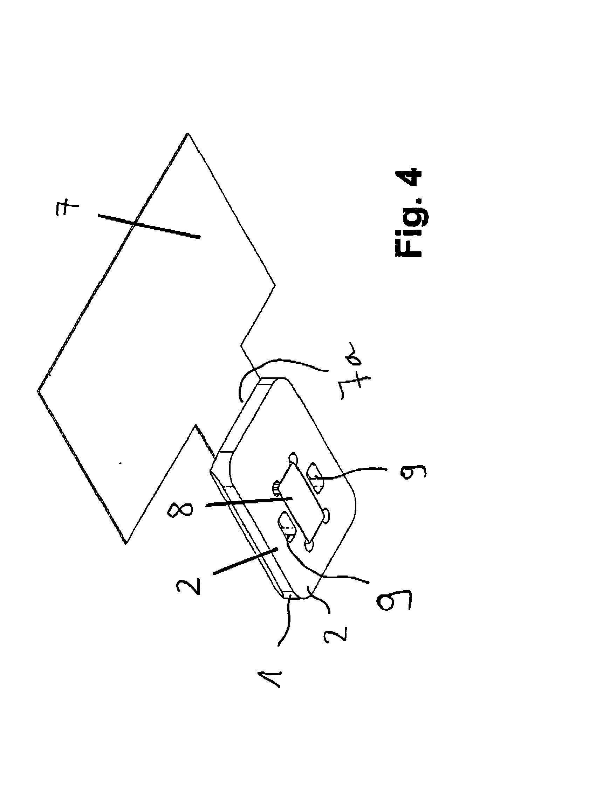

Fig. 3 und 4 zeigen, dass die Abfragelichtquelle 4 und der Detektor 5 auf oder in einem ebenen Bereich 7a einer Leiterplatte 7 angeordnet sind, wobei sich der Bereich 7a der Leiterplatte 7 auf oder im Gehäuse 1 erstreckt.

Die Abfragelichtquelle 4 ist als im Infraroten leuchtende Diode, nämlich als LED, ausgestaltet. Es sind zwei Abfragelichtquellen 4 vorgesehen, welche als im Infraroten leuchtende Dioden, nämlich als LEDs, ausgestaltet sind. Lateral zwischen den LEDs ist der Detektor 5 angeordnet. Der Detektor 5 ist als infrarotselektive Photodiode ausgestattet. Der Detektor 5 und die Abfragelichtquellen 4 sind als nichtorganische oder organische optische Elemente ausgestaltet.

Die Fig. 3 und Fig. 4 zeigen, dass die Leiterplatte 7 als starre Leiterplatte ausgestaltet ist.

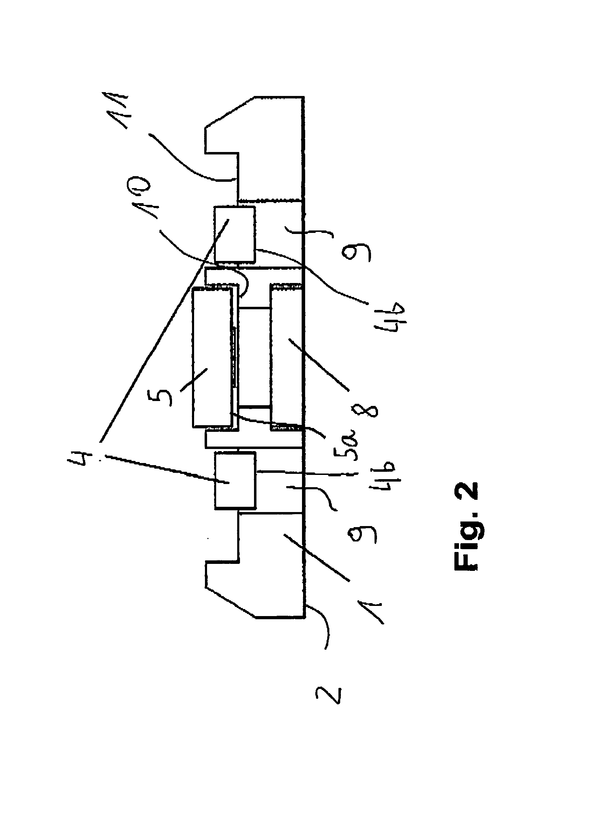

Die Fig. 1, 2 und 4 zeigen, dass dem Detektor 5 ein Filter 8 vorgeschaltet ist, welcher Primärficht der Abfragelichtquelle 4 ausblendet. Der Filter 8 ist zwischen dem Detektor 5 und der Anlagefläche 2 angeordnet und blendet Primärlicht aus, welches unmittelbar von den Abfragelichtqellen 4 kommt. Der Filter 8 lässt jedoch das Fluoreszenzlicht aus dem Gewebe zum Detektor 5 hindurch. Die Fig. 1 und 2 zeigen, dass die Abfragelichtquellen 4 in Kanälen 9 des Gehäuses 1 liegen. Der Detektor 5 liegt auf einem Absatz 10 im Gehäuse 1. Das Gehäuse 1 ist aus lichtundurchlässigem Kunststoff gefertigt.

Die Leiterplatte 7 bzw. der Bereich 7a liegt konkret auf einer ebenen Auflagefläche 11 im Gehäuse 1 auf. Die Abstrahlflächen 4b der Abfragelichtquellen 4 sind hierdurch im Wesentlichen parallel zur Detektionsfläche 5a des Detektors 5 orientiert.

Die Abfragelichtquelle 4 und der Detektor 5 sind durch Kupferkontakte 12 an die Leiterplatte 7 elektrisch leitend angebunden. Dies ist in Fig. 3 dargestellt. Die Abfragelichtquellen 4 strahlen Anregelicht 4a mit einer Wellenlänge aus dem Bereich 550 nm bis 1400 nm ab. Der Detektor 5 detektiert Antwortlicht 6 mit einer Wellenlänge aus dem Bereich 550 nm bis 1400 nm.

## Patentansprüche

1. Sensorkopf, umfassend ein Gehäuse (1) mit einer Anlagefläche (2), welche einem menschlichen oder einem tierischen Körper (3) zuwendbar ist, mit mindestens einer Abfragelichtquelle (4) und mit einem Detektor (5), wobei der Detektor (5) Antwortlicht (6) detektiert, welches durch die Abfragelichtquelle (4) erzeugbar ist,
**dadurch gekennzeichnet, dass** die Abfragelichtquelle (4) und der Detektor (5) auf oder in einem ebenen Bereich (7a) einer Leiterplatte (7) angeordnet sind, wobei sich der Bereich (7a) der Leiterplatte (7) auf oder im Gehäuse (1) erstreckt.

2. Sensorkopf nach Anspruch 1, **dadurch gekennzeichnet, dass** die Abfragelichtquelle (4) als im Infraroten leuchtende Diode, nämlich als LED, ausgestaltet ist.

3. Sensorkopf nach Anspruch 1 oder 2, **dadurch gekennzeichnet, dass** zwei Abfragelichtquellen (4) vorgesehen sind, welche als im Infraroten leuchtende Dioden, nämlich als LEDs, ausgestaltet sind, zwischen welchen der Detektor (5) angeordnet ist.

4. Sensorkopf nach einem der Ansprüche 1 bis 3, **dadurch gekennzeichnet, dass** der Detektor (5) als infrarotselektive Photodiode ausgestaltet ist.

5. Sensorkopf nach einem der voranstehenden Ansprüche, **dadurch gekennzeichnet, dass** der Detektor (5) und/ oder die Abfragelichtquellen (4) als nichtorganische oder organische optische Elemente ausgestaltet sind bzw. ist.

6. Sensorkopf nach einem der voranstehenden Ansprüche, **dadurch gekennzeichnet, dass** die Leiterplatte (7) als starre Leiterplatte ausgestaltet ist.

7. Sensorkopf nach einem der voranstehenden Ansprüche, **dadurch gekennzeichnet, dass** eine oder mehrere Abfragelichtquelle(n) (4) Anregelicht (4a) mit einer Wellenlänge aus dem Bereich 550 nm bis 1400 nm abstrahlt bzw. abstrahlen.

8. Sensorkopf nach einem der voranstehenden Ansprüche, **dadurch gekennzeichnet, dass** der Detektor (5) Antwortlicht (6) mit einer Wellenlänge aus dem Bereich 550 nm bis 1400 nm detektiert.

9. Sensorkopf nach einem der voranstehenden Ansprüche, **dadurch gekennzeichnet, dass** dem Detektor (5) ein Filter (8) vorgeschaltet ist, welcher Primärlicht der Abfragelichtquelle (4) ausblendet.

10. Pflaster, umfassend einen Sensorkopf nach einem der voranstehenden Ansprüche.
